Europäisches Patentamt

European Patent Office

Office européen des brevets

(11) Publication number: **0 223 960 B1**

# EUROPEAN PATENT SPECIFICATION

(45) Date of publication of patent specification: **15.07.92**    (51) Int. Cl.5: **C12P 7/64**

(21) Application number: **86113088.8**

(22) Date of filing: **23.09.86**

(54) **Process for the production of arachidonic acid-containing lipids.**

(30) Priority: **01.10.85 JP 218558/85**
**31.03.86 JP 73450/86**

(43) Date of publication of application:
**03.06.87 Bulletin  87/23**

(45) Publication of the grant of the patent:
**15.07.92 Bulletin  92/29**

(84) Designated Contracting States:
**AT BE CH DE FR GB IT LI NL SE**

(56) References cited:
**EP-A- 0 125 764**
**EP-A- 0 155 420**

**CHEMICAL ABSTRACTS, vol. 101, no. 19, 5th November 1984, page 539, no. 169707e, Columbus, Ohio, US; & JP-A-59 130 191 (AGENCY OF INDUSTRIAL SCIENCES AND TECHNOLOGY) 26-07-1984**

**CHEMICAL ABSTRACTS, vol. 98, no. 5, 31st January 1983, page 537, no. 33069f, Columbus, Ohio, US; & JP-A-57 144 986 (AGENCY OF INDUSTRIAL SCIENCES AND TECHNOLOGY) 07-09-1982**

(73) Proprietor: **LION CORPORATION**
**3-7, Honjo 1-chome**
**Sumida-ku Tokyo(JP)**

(72) Inventor: **Totani, Nagao**
**102 Kopo Meiwa 1-12, Nakacho 3-chome**
**Odawara-shi Kanagawa-ken(JP)**
Inventor: **Suzaki, Kazuhiko**
**17-34, Fujimigaoka 3-Chome Ninomiyamachi**
**Nakagun Kanagawa-ken(JP)**
Inventor: **Kudo, Toshihiro**
**306, 2-2, Minamigaoka 2-chome**
**Hadano-shi Kanagawa-ken(JP)**

(74) Representative: **Neidl-Stippler, Cornelia, Dr.**
**Rauchstrasse 2**
**W-8000 München 80(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

BACKGROUND OF THE INVENTION

Field of the Invention

This invention relates to a process for the production of arachidonic acid-containing lipids, and particularly to a process for the production of lipids containing arachidonic acid in high content by cultivating a specific species belonging to the genus Mortierella.

Prior Art of the Invention

Arachidonic acid is believed to be a precursor of prostaglandins, thromboxanes, prostacyclin, leukotrienes and the like which have various and strong physiological activities such as oxytocic and atonic activities, vasodilating activity and hypotensive activity, and it now attracts a good deal of public attention.

Arachidonic acid is widely present in the animal kingdom and has heretofore been isolated from the lipids extracted from adrenal gland, liver or sardines. However, since the content of arachidonic acid in these lipids is usually less than 5%, the yield per cell dry weight is only 0.2% or lower, and it is difficult to get the raw materials in a large scale, this extraction method cannot be useful one for the production of arachidonic acid.

On the other hand, many methods have been proposed for the production of arachidonic acid by cultivating various microorganisms capable of producing arachidonic acid. For instance, Japanese Patent Publication (unexamined) Nos. 64482/1977, 64483/1977 and 64484/1977 disclose a method for the production of arachidonic acid, in which an arachidonic acid-producing microorganism belonging to the genus Penicillium, Cladosporium, Mucor. Fusarium, Hormodendram, Aspergillus, or Rhodotorura, is cultivated in a medium containing a carbon source such as hydrocarbon or carbohydrate to collect arachidonic acid from the culture broth. However, the content of arachidonic acid in the lipids obtained by this method is only 7.5% or below and the yield of the acid per the dry weight of the cells is less than 1%.

It has been reported that some of the strains belonging to the genuses Entomophthora, Delacroixia, Conidiobolus, Pythium and Phytophthera which belong to Entomophthorales of Zygomycetes produced arachidonic acid-containing lipids, and the contents of the acid in the lipids were 27.1% based on the weight of whole fatty acids in E. extitialis, 19.1% in E. ignobilis and 18.8% in E. thaxteriana (D. Tyrrell, Canadian Journal of Microbiology, Vol. 13 (1967), pp. 755-760). It has also been reported that Mortierella renispora produced arachidonic acid-containing lipids, the contents of which in the mycelia were 4.8% and the content of arachidonic acid in the lipids was 26.7% (R.H. Haskins et al., Canadian Journal of Microbiology, Vol. 10 (1964), pp. 187-195) and that the red alga, Porphyridium cruentum produced arachidonic acid, the yield of which was less than 1% of the total dry weight of the cells (T.J. Ahern, Biotechnology and Bioengineering, Vol. XXV, pp. 1057-1070(1983)).

Further, it has been reported that Mortierella elongata cultured in a liquid medium containing yeast extract and malto extract produced 0.5 to 1.0 g of arachidonic acid per liter of the liquid medium and the content of arachidonic acid in the whole fatty acids was 30.1% (S. Yamada et al. Annual Conference of the Agricultural and Chemical Society of Japan, a summary of lectures, page 502, March 10,1986)

However, the contents of arachidonic acid in the total dry weight of the cells and in the lipids produced by these species as well as the yield of arachidonic acid per weight of medium used were not so high from the standpoint of practical use.

SUMMARY OF THE INVENTION

Accordingly, an object of this invention is to provide a process for the production of arachidonic acid-containing lipids by the cultivation of an arachidonic acid-producing microorganism, wherein the contents of arachidonic acid in the total dry weight of the cells as well as in the lipids extracted from the cells are so high that it is easy to collect and purify arachidonic acid and to obtain highly purified arachidonic acid in high yield.

The inventors of this invention studied the ability to produce arachidonic acid regarding the species of the genus Mortierella and found out that certain Mortierella species produce the lipids containing arachidonic acid in a high amount and that certain culture media can increase the total cell weight of the microorganism grown therein.

According to an aspect of this invention, there is provided a process for the production of arachidonic acid-containing lipids by cultivating a strain, of Mortierella species selected from the group consisting of Mortierella alpina, Mortierella bainieri, Mortierella elongata, Mortierella exigua, Mortierella minutissima, Mortierella verticillata, Mortierella hygrophila and Mortierella polycephala.

According to the preferred embodiment of this invention, the strain of Mortierella species is cultivated in a culture medium comprising a tuber.

## DETAILED DESCRIPTION OF THE INVENTION

Specific examples of a species which can advantageously be used in this invention include Mortierella alpina (IFO 8568, ATCC 16266, ATCC 32221, ATCC 42430), Mortierella bainieri (IFO 8569), Mortierella elongata (IFO 8570), Mortierella exigua (IFO 8571), Mortierella minutissima (IFO 8573), Mortierella verticillata (IFO 8575), Mortierella hygrophila (IFO 5941), and Mortierella polycephala (IFO 6335). All of these strains are mold and listed in the strain catalogues of the Institute of Fermentation, Osaka - (IFO), Japan and American Type Culture Collection (ATCC).

The present strains can be cultivated in a solid or liquid medium by static or stir culture with shaking or under aerated agitation.

According to one of the preferred embodiments of this invention, the present strains are cultivated in a culture medium comprising a tuber such as a potato, a taro, a sweet potato, a cassava, a yam or Jerusalem artichoke, with a potato being preferred. For preparing a solid medium, a tuber is cut into about 1 cm-cubes, added with 0 to 2 times, preferably 0 to 1 time water, boiled and crushed well, to which carbohydrate is added in an amount of 0 to 20%, preferably 2 to 10% and mixed well. If water is added in an amount of more than 2 times the weight of the tuber, it is impossible to prepare a solid medium. For preparing a liquid medium, 300 to 2000 g, preferably 400 to 1000 g of the tuber cut into about 1 cm-cubes is boiled in 1000 ml of water for about 20 minutes, filtered through a cloth and diluted with distilled water to obtain 1000 ml of an extract to which carbohydrate is added in an amount of 0 to 20%, preferably 2 to 10% before sterilization of the extract. Alternatively, carbohydrate separately sterilized may be added to the extract sterilized. Examples of the carbohydrate include glucose, fructose, saccharose, molasses, saccharified woods and starch hydrolyzates.

According to the other of the preferred embodiments of this invention, the present strains are cultivated in a culture medium comprising a tuber and a divalent metal ion such as $Ca^{2+}$ or $Mg^{2+}$. $Ca^{2+}$ is added in an amount of 0.02 to 2 g, preferably 0.05 to 1 g per 1 or kg of the medium and $Mg^{2+}$ in an amount of 0.01 to 5 g, preferably 0.02 to 2 g per 1 or kg of the medium.

Further, there may be added a nitrogen source such as ammonia, an ammonium salt, glutamic acid, aspartic acid or urea, an inorganic salt such as potassium, sodium, iron, zinc, copper or manganese salt, a trace element and other nutrients. There may also be used a medium comprising malt-extract, peptone, yeast extract, corn steep liquor or casamino acid with or without carbohydrate.

Initial pH of the culture medium is suitably in the range of 4.0 to 7.0. The cultivation is conducted at 10 to 33°C, preferably 20 to 30°C for 2 to 20 days.

The present strains grow under such aerobic condition and produce lipids most of which are contained within the cells. Therefore, the cells are separated from the culture fluid, crushed mechanically or physically and extracted with a solvent or supercritical carbon dioxide to obtain lipids containing arachidonic acid in high content.

The resulting lipids are subjected to conventional hydrolysis, esterification or interesterification to assay the content of arachidonic acid. Because of the high content of arachidonic acid in the lipids, it is possible to easily and economically purify arachidonic acid or its ester by solvent or chromatography fractionation or urea adduct separation method, as compared with the prior art. The maximum yield of arachidonic acid or its ester according to this invention reaches 28.7% based on the total dry weight of the cells which corresponds to 20 to 30 times the yield of the prior art method; the yield based on the weight of the culture medium, 2 to 13 times that of the prior art.

According to this invention, it is possible, to obtain arachidonic acid 30 times more on the base of the total dry weight of the cells than those obtained by the prior art process, or to obtain arachidonic acid-containing lipids in a yield (per the weight of the medium) 13 times higher than that of the prior art process.

Because of the high content of arachidonic acid in the lipids as well as in the medium, it becomes possible to purify arachidonic acid very easily and in a shortened time using a smaller culture tank to thereby supply highly purified arachidonic acid in a large scale at a low cost.

So far, prostaglandin related compounds, pharmacological activities of which are utilized or expected, are directly synthesized from arachidonic acid by a biochemical process using cyclooxygenase, which has an advantage in that it is unnecessary to remove various isomers unlike a chemical process. The process of this invention can provide highly purified arachidonic acid in a large scale at a low cost, which can contribute to a biochemical process for the production of the prostaglandin related compounds.

EXAMPLE 1

A potato (600 g) peeled and cut into cubes with an edge of 1 cm was boiled in 400 ml of water for 20 minutes and passed through No. 32 mesh (0.5 mm × 0.5 mm) to prepare potato paste (or slurry) which was mixed with 60 g of glucose and sterilized by autoclaving. Before cooled to room temperature, the paste was poured into 70 sterilized dishes of 80 mm in diameter to prepare a solid medium.

Mortierella alpina (IFO 8568), Mortierella alpina (ATCC 32221) and Mortierella elongata (IFO 8570) were inoculated in an amount of a platinum earpick into each of 30, 20 and 20 of the resulting dishes, respectively and incubated at 25°C for 20 days.

Mycelia grown on 20 dishes of each for IFO 8568 and ATCC 32221 were collected. As for the remaining ten dishes for IFO 8568 and 20 dishes for IFO 8570, mycelia and pellicle together were scraped and collected with a spatula. The mycelia - (and pellicle) thus collected were immediately dried, crushed with chloroform/methanol (2:1, v/v) in a mortar and subsequently extracted with chloroform/methanol (2:1, v/v). The lipids thus obtained were converted to methyl esters with sodium methoxide. The fatty acid composition of the esters was analyzed by gas chromatography to determine the content of arachidonic acid. The results are shown in Table 1.

The same procedures except that 735 mg of $CaCl_2 \cdot 2H_2O$ was added to 1 kg of the paste, were repeated to prepare 20 dishes of a solid medium. IFO 8568 were inoculated into the dishes and incubated at 25°C for 20 days. Similarly, mycelia and pellicle were collected and treated. The results are also shown in Table 1.

Malto agar medium (22.5 g) and Sabouraud agar medium (32.5 g) (both produced by NISSUI Pharmaceutical Co., Japan) were each added to 500 ml of distilled water, sterilized by autoclaving and poured into 25 dishes, respectively to prepare agar media. Mortierella alpina (IFO 8568) were inoculated in an amount of a platinum earpick into the dishes and incubated at 25°C for 20 days. Similarly, mycelia were collected, dried and treated. The results are shown in Table 1.

The content of arachidonic acid in the lipids of the mycelia grown on the potato media were higher than that in the lipids of the pellicle, while the cell yield of the mycelia was lower than that of the pellicle. The yield of arachidonic acid in the mycelia was about 5 g per 1 kg of the medium and that in the mycelia and the pellicle was more than 10 g, which was 5 to 13 times the yield in the liquid culture of the Suntory-Kyoto method (0.5 to 1.0 g/l).

The addition of calcium chloride increased the yields of the cells and the lipids to thereby increase the yield of arachidonic acid by 27%, which showed a remarkable effect of calcium chloride.

## Table 1

| Strain | Medium | Part | Dry weight of the cells per the medium weight (g/kg) | Methyl ester content per the dry weight of the cells (%) | Methyl arachidonate content in the methyl esters (%) | Methyl arachidonatic content in the dry weight of the cells | Methyl arachidonate yield per the medium weight (g/kg) |
|---|---|---|---|---|---|---|---|
| IFO 8568 | Potato | Mycelia | 36.5 | 23.2 | 67.4 | 15.6 | 5.7 |
|  |  | Mycelia + Pellicle | 85.8 | 26.6 | 45.1 | 12.0 | 10.3 |
|  | Potato CaCl$_2$ | Mycelia + Pellicle | 95.9 | 27.8 | 49.2 | 13.7 | 13.1 |
|  | Malto agar | Mycelia | 1.08 | 33.7 | 78.8 | 26.6 | 0.287 |
|  | Sabouraud agar | Mycelia | 9.76 | 6.9 | 31.1 | 2.1 | 0.205 |
| ATCC 32221 | Potato | Mycelia | 28.7 | 29.2 | 64.5 | 18.8 | 5.4 |
| IFO 8570 | Potato | Mycelia + Pellicle | 84.4 | 33.3 | 28.8 | 9.6 | 8.1 |

EXAMPLE 2

Extracts obtained from 100 g, 300 g or 500 g of potato were added with 30 g of glucose and diluted with distilled water to 500 ml, respectively. The resulting culture media were poured into 250 ml L-shaped tubes and sterilized. Mortierella alpina (IFO 8568) were inoculated into the media and incubated at 25°C for 20 days under shaking. The cells were collected by centrifugation, washed, dried and treated in a similar manner as in EXAMPLE 1. The results are shown in Table 2.

The higher the concentration of potato extracts, the greater the yield of arachidonic acid.

Table 2

| Strain | Medium (Potato) (g/l) | Dry weight of the cells per the medium volume (g/l) | Methyl ester content per the dry weight of the cells (%) | Methyl arachidonate content in the methyl esters (%) | Methyl arachidonate content in the dry weight of the cells (%) | Methyl arachidonate yield per the medium volume (g/l) |
|---|---|---|---|---|---|---|
| IFO 8568 | 200 | 6.48 | 36.5 | 42.3 | 15.4 | 0.998 |
| | 600 | 14.8 | 29.0 | 39.7 | 11.5 | 1.70 |
| | 1000 | 18.0 | 30.9 | 37.8 | 11.7 | 2.11 |

EXAMPLE 3

An extract obtained from 600 g of potato was added with 60 g of glucose and diluted with distilled water to 1000 ml which was poured into four L-shaped tubes in 250 ml each and sterilized. The aqueous solutions of 185 mg of $CaCl_2 \cdot 2H_2O$, 100 mg and 515 mg of $MgCl_2 \cdot 6H_2O$ dissolved in 1 ml of water and sterilized were added to the three L-shaped tubes, respectively. Mortierella alpina (IFO 8568) were inoculated into the four tubes and incubated at 25°C for 20 days under shaking. The cells were collected by centrifugation, washed, dried and treated in a similar manner as in EXAMPLE 1. The results are shown in Table 3.

Table 3

| Medium | Dry weight of the cells per the medium volume (g/l) | Methyl ester content per the dry weight of the cells (%) | Methyl anachidonate content in the methyl esters (%) | Methyl arachidonate content per the dry weight of the cells (%) | Methyl arachidonate yield per the medium volume (g/l) | Increase of the yield of methyl arachidonate (%) |
|---|---|---|---|---|---|---|
| $CaCl_2 \cdot 2H_2O$ 185 mg/250 ml | 16.8 | 27.7 | 42.7 | 11.8 | 1.99 | 17.1 |
| $MgCl_2 \cdot 6H_2O$ 100 mg/250 ml | 18.2 | 26.9 | 40.7 | 10.9 | 1.99 | 17.1 |
| $MgCl_2 \cdot 6H_2O$ 515 mg/250 ml | 13.7 | 36.3 | 35.5 | 12.9 | 1.77 | 4.1 |
| None | 14.8 | 29.0 | 39.7 | 11.5 | 1.70 | 0 |

Table 3 shows that $Ca^{2+}$ and $Mg^{2+}$ increased the yields of methyl arachidonate, although the increases were lower than those obtained by the use in the solid media of EXAMPLE 1. The contents of methyl arachidonate in the dry weight of the cells were almost same in the four media.

EXAMPLE 4

An extract obtained from 200 g of potato and 20 g of glucose were diluted with distilled water, to 1000 ml and adjusted to pH5.6. The resulting medium (200 ml) was charged in a 500-ml Sakaguchi flask, into which Mortierella alpina (IFO 8568) and Mortierella elongata (IFO 8570) in an amount of a platinum earpick were inoculated and incubated at 25°C for 6 days under shaking. The resulting cells were immediately collected by centrifugation at 6000 rpm, dewatered with filter paper and weighed. One portion was used to determine the dry weight of the cells and the remaining portion was crushed with chloroform/methanol (2:1, v/v) in a mortar and extracted with chloroform/methanol (2:1, v/v). The lipids extracted was converted into methyl esters by sodium methoxide. The fatty acid compositions were analyzed by gas chromatography to thereby determine the content of arachidonic acid in the lipids. The results are shown in Table 4.

Table 4

| Strain | Dry weight of the cells per the medium volume (g/l) | Methyl ester content per the dry weight of the cells (%) | Methyl arachidonate content in the methyl esters (%) | Methyl arachidonate content per the dry weight of the cells (%) |
|---|---|---|---|---|
| Mortierella alpina (IFO 8568) | 6.14 | 29.6 | 36.9 | 10.9 |
| Mortierella elongata (IFO 8570) | 8.02 | 39.4 | 15.8 | 6.2 |

As described earlier, Haskins et al. have reported that Mortierella renispora produced lipids in an amount of 4.8% per the dry weight of the cells and that the content of arachidonic acid was 26.7% of the lipids, which corresponded to 1.28% (= 26.7% $\times$ 4.8%) expressed in the content of arachidonic acid per the dry weight of the cells. According to this invention, the content of arachidonic acid per the dry weight of the cells was 10.9% for alpina and 6.2% for elongata which were about 8 and 5 times that of the Haskins

10

method, respectively and show that this invention is higher in the productivity than the Haskins method.

EXAMPLE 5

An extract obtained from 400 g of potato was added with 40 g of glucose and 40 g of agar and diluted with distilled water to 2000 ml (pH 5.6) which was then sterilized by autoclaving and poured into 100 sterilized dishes to prepare agar media. Mortierella alpina (IFO 8568) and Mortierella elongata (IFO 8570) in an amount of a platinum earpick were inoculated into every 50 dishes, respectively and incubated at 25°C for 10 days. After the cultivation, white cotton-like mycelia on the culture media were collected with a spatula and treated in a similar manner as in EXAMPLE 4. The results are shown in Table 5.

Similarly, Mortierella alpina (ATCC 16266, ATCC 32221, ATCC 42430), Mortierella bainieri - (IFO 8569), Mortierella exigua (IFO 8571), Mortierella minutissima (IFO 8573), Mortierella verticillata (IFO 8575), Mortierella hygrophila (IFO 5941) and Mortierella polycephala (IFO 6335) were cultivated. Analytical results of methyl esters obtained from the extracted lipids are shown in Table 5.

Table 5

| Strain | Methyl ester content per the dry weight of the cells (%) | Methyl arachidonate content in the methyl esters (%) | Methyl arachidonate content per the dry weight of the cells (%) | Productivity (vs. Haskins method) / Productivity (vs. other prior art method) |
|---|---|---|---|---|
| Mortierella alpina IFO 8568 | 35.8 | 80.2 | 28.7 | 22 times / 29 " |
| Mortierella alpina ATCC 16266 | 37.0 | 64.8 | 24.0 | 19 " / 24 " |
| Mortierella alpina ATCC 32221 | 34.7 | 70.6 | 24.5 | 19 " / 25 " |
| Mortierella alpina ATCC 42430 | 27.9 | 80.1 | 22.3 | 17 " / 22 " |
| Mortierella bainieri IFO 8569 | 38.6 | 28.0 | 10.8 | 8 " / 11 " |
| Mortierella elongata IFO 8570 | 46.2 | 35.7 | 16.5 | 13 " / 17 " |
| Mortierella exigua IFO 8571 | 14.3 | 37.6 | 5.4 | 4 " / 5 " |
| Mortierella minutissima IFO 8573 | 33.6 | 45.5 | 15.3 | 12 " / 15 " |
| Mortierella verticillata IFO 8575 | 33.0 | 42.3 | 14.0 | 11 " / 14 " |
| Mortierella hygrophila IFO 5941 | 21.5 | 30.3 | 6.5 | 5 " / 7 " |
| Mortierella polycephala IFO 6335 | 14.5 | 47.2 | 6.8 | 5 " / 7 " |

The content of methyl arachidonate per the dry weight of the cells of Mortierella alpina was 22 and 29 times higher than the Haskins method and the other prior art methods, respectively, which shows significantly high productivity of the process of this invention.

EXAMPLE 6

45 g of malto-agar medium (produced by NISSUI Pharmaceutical Co.) was added to 1000 ml of distilled water and sterilized at 121°C for 15 minutes by autoclaving. The resulting medium was poured into 50 sterilized dishes of 80 mm in diameter. The dishes were divided into 5 groups consisting of 10 dishes. Each dish of the 5 groups was inoculated with Mortierella alpina (IFO 8568), Mortierella bainieri (IFO 8569),

12

Mortierella elongata (IFO 8570), Mortierella minutissima (IFO 8573) and Mortierella verticillata (IFO 8575) in an amount of a platinum earpick, respectively and incubated at 25°C for 10 days. After the cultivation, the white mycelia on the medium were collected with a spatula and treated in a similar manner as in EXAMPLE 4. The results are shown in Table 6.

Similarly, Mortierella alpina ATCC 16266, ATCC 32221 and ATCC 42430 were incubated. The analytical results of methyl esters obtained from the lipids extracted are also shown in Table 6.

Table 6

| Strain | Methyl ester content per the dry weight of the cells (%) | Methyl arachidonate content in the methyl esters (%) | Methyl arachidonate content per the dry weight of the cells (%) |
|---|---|---|---|
| Mortierella alpina IFO 8568 | 33.7 | 78.8 | 26.6 |
| Mortierella alpina ATCC 16266 | 32.4 | 68.5 | 22.2 |
| Mortierella alpina ATCC 32221 | 37.5 | 70.3 | 26.4 |
| Mortierella alpina ATCC 42430 | 36.5 | 70.1 | 25.6 |
| Mortierella bainieri IFO 8569 | 29.5 | 26.4 | 7.8 |
| Mortierella elongata IFO 8570 | 24.8 | 30.0 | 7.4 |
| Mortierella minutissima IFO 8573 | 15.4 | 53.0 | 8.2 |
| Mortierella verticillata IFO 8575 | 14.0 | 50.9 | 7.1 |

13

EXAMPLE 7

32.5 g of Sabouraud agar medium (produced by NISSUI Pharmaceutical Co.) was added to 500 ml of distilled water and sterilized at 121°C for 15 minutes by autoclaving. The resulting medium was poured into 25 sterilized dishes of 80 mm in diameter. Mortierella alpina (ATCC 42430) was inoculated into the medium in an amount of a platinum earpick and incubated at 25°C for 10 days. After the cultivation, white mycelium on the medium was collected with a spatula and treated in a similar manner as in EXAMPLE 4. The results are shown in Table 7.

Table 7

| Strain | Methyl ester content per the dry weight of the cells (%) | Methyl arachiodo-nate content in the methyl esters (%) | Methyl arachidonate content per the dry weight of the cells (%) |
|---|---|---|---|
| Mortierella alpina ATCC 42430 | 23.3 | 65.1 | 15.2 |

For the purification of arachidonate 50 mg of methyl esters obtained by the esterification of the total lipids produced by Mortierella alpina was subjected to reversed phase thin layer chromatography (RP-18F produced by Merck, methanol/acetonitrile 1:1, v/v). A band at Rf 0.41 was scraped and recovered to give 35 mg of methyl arachidonate having the purity of 95.9% (the remaining 4.1% being methyl γ-linolenate).

Identification of Methyl Arachidonate

Identification of methyl arachidonate (methyl eicosa -5, 8, 11, 14-tetraenoate, molecular weight 318.5) isolated from the cells of Mortierella species was conducted in terms of the following 5 items.

(i) Elemental analysis

Methyl arachidonate having the purity of 95.9% - (the remaining 4.1% being methyl γ-linolenate) was analyzed.

Found: C : 79.34%, H 11.21%

Calcd: C : 79.15%, H 10.77%

(ii) Gas chromatography

Retention times of the sample on DEGS 15% - (column temperature 190°C), SE-30 (column temperature 170°C) and OV101 (column temperature 170°C) agreed well with those of the authentic sample.

(iii) Gas-mass spectrum analysis

Mass fragment pattern obtained by separating the sample on DEGS 10% (column temperature 200°C) and ionizing the corresponding peak at 70 e$_\nu$ resembled well with that of the authentic sample, wherein the parent peak appeared at m/e 318. Fragment signals greater than m/e 200 were determined at 5 times sensitivity at which the signals of m/e 0-200 were determined.

(iv) H-NMR spectrum

H-NMR spectrum for the sample resembled very well with that for the authentic sample. Taking the strength of three methyl protons in methyl ester group at δ value near 3.6 ppm as the standard, there were 8 protons (5.0-5.7 ppm) which are directly bonded to a double bond nucleus and 6 protons (2.6-3.3 ppm) of methylene between double bonds, which supported the chemical structure of methyl tetraenoate.

(v) C$^{13}$-NMR

Each of signal patterns around 15-35 ppm derived from methylene carbon, around 50 ppm derived from methyl ester carbon, around 130 ppm derived from carbons forming a double bond nucleus resembled well with those of the authentic sample. Accordingly, it was confirmed that the sample was not an isomer of methyl arachidonate in terms of positions of four double bonds in arachidonate.

**Claims**

1. A process for the preparation of a lipid rich in its content of arachidonic acid which comprises the culturing of a fungus belonging to the genus of Mortierella in a growth medium, the collection of the fungal body and the isolation of the arachidonic acid, characterised therein that a lipid rich species selected from the group consisting of Mortierella alpina, Mortierella bainieri, Mortierella elongata, Mortierella exigua, Mortierella minutissima, Mortierella verticillata, Mortierella hygrophilia and Mortierella polycephala is cultured.

2. A process as claimed in Claim 1, characterised therein that said Mortierella strain is Mortierella alpina.

3. A process as claimed in Claim 1 or Claim 2, characterised therein that said growth medium contains tubers as a main constituent.

4. A process as claimed in Claim 3, characterised therein that said tubers are selected from the group consisting of potato, taro, sweet potato, cassava, yam and Jerusalem artichoke.

5. A process as claimed in Claim 4, characterised therein that said tubers are potatoes.

6. A process as claimed in Claim 4, characterised therein that said growth medium is a solid medium comprising one part by weight of potato and 0 to 2 parts by weight of water.

7. A process as claimed in Claim 4, characterised therein that said growth medium is liquid and comprises an extract of 0.3 to 2 parts by weight of potato and one part by weight of water.

8. A process as claimed in Claim 6 or Claim 7, characterised therein that said growth medium further comprises 0 to 20 % by weight of carbohydrate based on the weight of the whole medium.

9. A process as claimed in any one of the preceeding Claims characterised therein that said growth medium further comprises a divalent ion.

10. A process as claimed in any one of the preceeding Claims characterised therein that said divalent ion is $Ca^{2+}$ or $Mg^{2+}$.

11. A process as claimed in any one of the preceeding Claims characterised therein that said divalent ion is contained in an amount of 0.01 to 5 g per kg in said growth medium.

12. A process as claimed in any one of the preceeding Claims characterised therein that the culturing is continued in a growth medium at an initial pH of from 4.0 to 7.0 at a temperature in the range of from 10 to 33°C for a period of from 2 to 20 days.

13. A process for the preparation of lipid rich in its content of arachidonic acid as claimed in any one of the preceeding Claims, characterised therein that the fungi cultured is selected from the group consisting of Mortierella alpina IFO 8568, Mortierella bainieri IFO 8569, Mortierella elongata IFO 8570, Mortierella exigua IFO 8571, Mortierella hygrophila IFO 5941, Mortierella minutissima IFO 8573, Mortierella polycephala IFO 6335, Mortierella verticillata IFO 8575.

**Revendications**

1. Procédé de préparation d'un lipide riche en acide arachidonique qui consiste à cultiver un fungus appartenant à l'espèce Mortierella dans un milieu de croissance, à recueillir la masse fongique et à isoler l'acide arachidonique, caractérisé en ce que l'on cultive une souche riche en lipide choisie dans le groupe suivant : Mortierella alpina, Mortierella bainieri, Mortierella elongata, Mortierella exigua, Mortierella minutissima, Mortierella verticillata, Mortierella hygrophilia et Mortierella polycephala.

2. Procédé selon la revendication 1, caractérisé en ce que la souche de Mortierella précitée est la Mortierella alpina.

3. Procédé selon la revendication 1 ou 2, caractérisé en ce que le milieu de croissance précité contient des tubercules comme constituant principal.

4. Procédé selon la revendication 3, caractérisé en ce que les tubercules précités sont choisis dans le groupe constitué de la pomme de terre, du taro, de la patate douce, du manioc, de l'igname et du topinambour.

5. Procédé selon la revendication 4, caractérisé en ce que les tubercules précités sont des pommes de terre.

6. Procédé selon la revendication 4, caractérisé en ce que le milieu de croissance précité est un milieu solide comprenant une partie en poids de pomme de terre et 0 à 2 parties en poids d'eau.

7. Procédé selon la revendication 4, caractérisé en ce que le milieu de croissance précité est liquide et comprend un extrait de 0,3 à 2 parties en poids de pomme de terre et une partie en poids d'eau.

8. Procédé selon la revendication 6 ou 7, caractérisé en ce que le milieu de croissance précité comprend par ailleurs 0 à 20 % en poids d'un carbohydrate sur base du poids de l'ensemble du milieu.

9. Procédé selon l'une quelconque des revendications précédentes, caractérisé en ce que le milieu de croissance précité comprend par ailleurs un ion bivalent.

10. Procédé selon l'une quelconque des revendications précédentes, caractérisé en ce que l'ion bivalent précité est $Ca^{2+}$ ou $Mg^{2+}$.

11. Procédé selon l'une quelconque des revendications précédentes, caractérisé en ce que l'ion bivalent précité est présent à raison de 0,01 à 5 g par kg dans le milieu de croissance précité.

12. Procédé selon l'une quelconque des revendications précédentes, caractérisé en ce que la culture est effectuée dans un milieu de croissance à un pH initial de 4,0 à 7,0 et à une température de l'ordre de 10 à 33°C pendant une période de 2 à 20 jours.

**13.** Procédé de préparation d'un lipide riche en acide arachidonique selon l'une quelconque des révendication précédentes, caracterisé en ce que l'on cultivé une souche choisie dans le groupe suivant: Mortierella alpina IFO 8568, Mortierella bainieri IFO 8569, Mortierella elongata IFO 8570, Mortierella exigua IFO 8571, Mortierella hygrophila IFO 5941, Mortierella minutissima IFO 8573, Mortierella polycephala IFO 6335 et Mortierella verticillata IFO 8575.

## Patentansprüche

**1.** Verfahren zur Herstellung von Arachidonsäure enthaltenden Lipiden, wobei ein Pilz vom Genus Mortierella in einem Wachstumsmedium gezüchtet, das Pilzmaterial gesammelt und die Arachidonsäure isoliert werden, dadurch gekennzeichnet, daß eine lipidhaltige Spezies ausgewählt aus Mortierella alpina, Mortierella bainieri, Mortierella elongata, Mortierella exigua, Mortierella minutissima, Mortierella verticillata, Mortierella hygrophilia und Mortierella polycephala gezüchtet wird.

**2.** Verfahren gemäß Anspruch 1, dadurch gekennzeichnet, daß der Mortierella-Stamm Mortierella alpina ist.

**3.** Verfahren gemäß Anspruch 1 oder 2, dadurch gekennzeichnet, daß das Wachstumsmedium als Hauptbestandteil Knollen enthält.

**4.** Verfahren gemäß Anspruch 3, dadurch gekennzeichnet, daß die Knolle aus Kartoffel, Taro, Süßkartoffel, Cassava (Maniok), Yamswurzel und Jerusalem-Artischocke ausgewählt ist.

**5.** Verfahren gemäß Anspruch 4, dadurch gekennzeichnet, daß die Knollen Kartoffeln sind.

**6.** Verfahren gemäß Anspruch 4, dadurch gekennzeichnet, daß das Wachstumsmedium ein Festmedium ist und einen Gewichtsteil Kartoffel und 0 bis 2 Gewichtsteile Wasser aufweist.

**7.** Verfahren gemäß Anspruch 4, dadurch gekennzeichnet, daß das Wachstumsmedium flüssig ist und einen Extrakt aus 0,3 bis 2 Gewichtsteilen Kartoffel und einem Gewichtsteil Wasser aufweist.

**8.** Verfahren gemäß Anspuch 6 oder 7, dadurch gekennzeichnet, daß das Wachstumsmedium ferner 0 bis 20 Gew.% Carbohydrate, bezogen auf das Gewicht des Gesamtmediums, aufweist.

**9.** Verfahren gemäß irgendeinem der vorangegangenen Ansprüche, dadurch gekennzeichnet, daß das Wachstumsmedium ferner ein divalentes Ion aufweist.

**10.** Verfahren gemäß irgendeinem der vorangegangenen Ansprüche, dadurch gekennzeichnet, daß das divalente Ion $Ca^{2+}$ oder $Mg^{2+}$ ist.

**11.** Verfahren gemäß irgendeinem der vorangegangenen Ansprüche, dadurch gekennzeichnet, daß das divalente Ion in einer Menge von 0,01 bis 5 g/kg Wachstumsmedium vorliegt.

**12.** Verfahren gemäß irgendeinem der vorangegangenen Ansprüche, dadurch gekennzeichnet, daß die Züchtung in einem Wachstumsmedium bei einem Anfangs-pH von 4,0 bis 7,0 bei einer Temperatur im Bereich von 10 bis 33°C über einen Zeitraum von 2 bis 20 Tagen erfolgt.

**13.** Verfahren zur Herstellung von Arachidonsäure enthaltenden Lipiden gemäß irgendeinem der vorangegangenen Ansprüche, dadurch gekennzeichnet, daß der gezüchtete Pilz ausgewählt ist aus Mortierella alpina IFO 8568, Mortierella bainieri IFO 8569, Mortierella elongata IFO 8570, Mortierella exigua IFO 8571, Mortierella hygrophila IFO 5941, Mortierella minutissima IFO 8573, Mortierella polycephala IFO 6335, Mortierella verticillata IFO 8575.